# EUROPEAN PATENT SPECIFICATION

(11) **EP 1 965 733 B1**
(45) Date of publication and mention of the grant of the patent: **20.02.2013**
(21) Application number: 06828357.1
(22) Date of filing: 18.12.2006
(51) Int. Cl.: A61L 27/36

(54) **BIOLOGICAL ARTIFICIAL CORNEA AND METHOD OF MAKING**
BIOLOGISCHE KÜNSTLICHE HORNHAUT UND HERSTELLUNGSVERFAHREN
CORNEE BIOLOGIQUE ARTIFICIELLE ET PROCEDE POUR LA PRODUIRE

(30) Priority: 20.12.2005 CN 200510120794
(43) Date of publication of application: 10.09.2008
(73) Proprietor: Grandhope (GD) Biotech Co., Ltd., Guangzhou 510663 (CN)
(72) Inventor: XU, Guofeng, Guangzhou 510630 (CN)
(74) Representative: Korga, Leokadia
(86) International application number: PCT/CN2006/003444
(87) International publication number: WO 2007/071169

(56) References cited:
- WO-A1-00/35374
- WO-A1-89/04153
- WO-A1-94/17851
- WO-A1-03/024496
- CN-A- 1 473 551
- CN-A- 1 579 342
- US-A- 4 793 344

## Description

The present invention relates to a medical prosthesis for human implantation, and in particular, to a device for reconstructing a damaged cornea.

Description of the Related Art

Loss of sight caused by corneal damage or pathological changes of the cornea is one of the most common ophthalmologic diseases, and the current treatment method relies on transplantation of a cornea donated from a cadaver. However, transplantation of a cornea not only has difficulties such as securing the source of donation, but immunological rejection often leads to failures in the transplantation. Accordingly, scientists have attempted to use animal corneas to treat corneal diseases in humans, including studies performed on the direct transplantation of animal corneas. However, such direct animal corneal transplantations were unsuccessful because of immunological rejection. Additional research on preparations of artificial corneas from animal corneas by low-temperature freezing and simple sterilization treatment were also unsuccessful because the elimination of antigens was not complete and the patients' bodies could not accept the transplants due to poor tissue compatibility.

WO94/17851 discloses a two-layer composite material composed of a thin layer of corneal tissue or collagen and a hydrogel for use for the construction of an artificial cornea. Corneal tissue can be obtained from rabbit or bovine eyes. The corneal collagen can be crosslinked. The hydrogel might be adhered to the layer of corneal collagen by crosslinking.

Thus, there still remains a need for an effective artificial cornea that can be harvested from animal corneas.

It is an object of the present invention to provide safe and reliable biological artificial corneas having high biocompatibility, stability, which can be degraded and absorbed, and which are capable of inducing cornea regeneration.

It is another object of the present invention to provide a method of preparing such an artificial cornea.

In order to accomplish the objects of the present invention, the present invention provides an artificial cornea for implantation into a human body which is made by a method that includes the steps of providing a natural animal cornea that has a substrate, crosslinking and fixing the substrate, minimizing the antigens from the substrate, and coupling an active layer to the substrate.

FIG. 1 is a perspective view of an artificial cornea according to one embodiment of the present invention.

FIG. 2 is a cross-sectional view of the artificial cornea of FIG. 1.

The following detailed description is of the best presently contemplated modes of carrying out the invention. This description is not to be taken in a limiting sense, but is made merely for the purpose of illustrating general principles of embodiments of the invention. The 5 scope of the invention is best defined by the appended claims.

The present invention provides a biological artificial cornea having a substrate made of an animal cornea that is crosslinked and fixed with a fixative, treated lo minimize antigens, and then coated with a surface layer containing an active layer.

Animal corneas are easily degraded or decomposed by microorganisms, so that crosslinking and fixation with a fixative is required. Conventionally, glutaraldehyde is utilized as a fixative, but glutaraldehyde produces toxic radicals. Aldehydes undergo crosslinking with proteins through the acetal reaction and toxic aldehydes are released when the crosslinked products are degraded, so that products fixed with an aldehyde have long-term residual toxicity. When epoxides, diamides, diisocyanates or carbodiimides are utilized as fixatives in place of aldehydes, this toxicity problem can be eliminated. For example, when an epoxide is utilized to replace aldehyde-type fixatives, a ring-opening, crosslinking reaction occurs readily because epoxides are unstable, but the crosslinking product can be made very stable and not easily degraded by controlling the reaction condition. It is slowly degraded into polypeptides and amino acids and absorbed only when tissue growth and regeneration begin to devour it by secreting kallikrein, fibrinolysin and glucocorticoid hormone to help collagenase in the degradation. Such kind of passive degradation and tissue regeneration are occurring simultaneously which is beneficial to tissue regenerative repair while having no residual toxicity of aldehydes. According to modem immunological theory, the antigenicity of animal tissues stems mainly from active groups located at specific sites and in specific conformations, and these active groups include -OH, -NH₂, -SH, etc. The specific conformations result mainly from some specific hydrogen bonding formed by spiral protein chains. The specific sites and conformations are called antigen determinants. One or more active reagents (e.g., acid anhydrides, acyl chlorides, amides, epoxides, etc.) that react readily with these groups are utilized to bond with and block these groups when treating animal corneas so that the antigens can be effectively eliminated. Simultaneously, reagents with strong hydrogen bonding (e.g., guanidine compounds) are utilized to replace the hydrogen bonding that gives the specific configurations so that the configurations are altered and the antigenicity is effectively eliminated.

Method

A method of preparing the biological artificial corneas according to the present invention comprises the following steps, using natural animal corneas as the substrate:

1. Selection of materials: Fresh animal eyeballs are collected. The corneal material is preferably transparent.

2. Pretreatment: Animal corneas are excised and neatly trimmed. The corneas are placed in a preserving solution and frozen at between -18 and - 4 C for 24-28 h, and then removed, thawed and soaked in a surfactant solution for 16-20 hours, or soaked in a pankrin solution for 2-4 hours, followed by washing, and if necessary washing for 10-20 minutes with ultrasound.

3. Fixation: The collagen molecules in the substrate are crosslinked and fixed using a non-aldehyde fixative, as described in greater detail hereinbelow.

4. Minimizing antigens: An active reagent is utilized to block the specific active groups such as -OH, -NH₂, -SH, etc., in the proteins of the substrate, and a reagent with strong hydrogen bonding power is utilized to replace the specific hydrogen bonding in the spiral chains of the protein molecules in the substrate and alter its specific configuration.

5. Coupling of active layer: An active surface layer containing a specific polypeptide or glucosaminoglycan capable of adhering to growth factors is incorporated on the surface layer using a coupling agent.

Surfactant

The surfactant in step 2 of the above method can be Triton X-100, sodium cholate, hydroxymethyl aminomethane (Tris), sodium dodecyl sulfate (SDS) or CHAPS. The pankrin can be pepsin, trypsin or a mixture of the two enzymes.

Preserving Solution

The preserving solution in step 2 of the above method can be an artificial tears solution, physiological saline solution, glycerol or a mixed solution of glycerol and artificial tears.

Fixative

The fixative applied in step 3 of the above method can be a reagent that crosslinks easily with protein molecules and is one or two reagents selected from epoxides, diacyl diamides, diisocyanates, polyethylene glycol or carbodiimides. This fixative may be an epoxy compound that has a hydrocarbon backbone, that is water-soluble, and which does not contain an ether or ester linkage in its backbone. This fixative is described in U.S. Patent No.6,106,555. Examples include an epoxide, a diamide, a diisocyanate, or a carbodiimide, in that the epoxide may be a monocyclic epoxide, or a bicyclic epoxide, or it may be a low poly (epoxide) (such as low poly (ethylene oxide), poly (propylene oxide) or a glycidyl ether). The epoxide may be a monocyclic epoxide of the formula 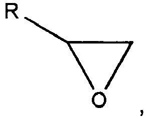 or a dicyclic epoxide of the formula: 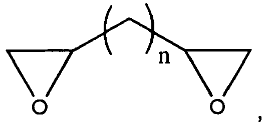 where R = - CₙH₂ₙ₊₁, and n=0-10, and may also be a lower polyepoxide such as polypropylene oxide.

Active Reagents

The active reagents in step 4 of the above method may be low molecular weight organic acid anhydrides, acyl chlorides, acylamides or monocyclic oxides, and the reagents having strong hydrogen bonding power are guanidine compounds.

Active Layer

The active layer in step 5 of the above method can be an active component such as a polypeptide or glycosaminoglycan. One example of a polypeptides is the polypeptide obtained from the condensation of 16 lysines (K16), glycine (G), arginine (R), asparagic acid (D), serine (S), praline (P) and cysteine (C), and sequence of the composition is K16-G-R-G-D-S-P-C. The glycosaminoglycan can be hyaluronic acid, chondroitin sulfate, dermatan sulfate, heparin, acetylheparin sulfate or keratan sulfate. These polypeptides or glycosaminoglycans exhibit a broad, spectrum adherence and enriching effects for growth factors or activate undifferentiated cells to perform oriented differentiation so that they are capable of exercising the function of inducing regenerative repair of organic tissues. Examples of growth factors for blood vessels that can adhere to and accumulate include vascular endothelial growth factor (VEGF), fibroblast growth factor (FGF), platelet-derived growth factor (PDGF-bb) and vascular permeability factor (VPF).

Coupling Agent for Active Layer

The coupling agent utilized for coupling the polypeptide or the glucosaminoglycan in step 5 of the above method may be a diacyl diamide, diacid anhydride, diepoxide or other bifunctional reagents capable of having a condensation reaction with -NH₂, -OH and -COOH.

The present invention provides the following advantages. The composition and the three-dimensional structure of the artificial cornea are very similar to those of a human cornea while having no immunogenicity; it can induce and promote cornea regeneration while being degraded correspondingly with cornea regeneration, and the rate of degradation can be regulated to coincide with the rate of cornea regeneration by controlling the crosslinking condition. The physical and mechanical properties of the artificial cornea are close to those of a human cornea having stable morphology and good flexibility while the cornea can be finished into various curvatures, and it does not swell in water, thereby making it an ideal substrate or support for reconstructing corneas.

Example 1

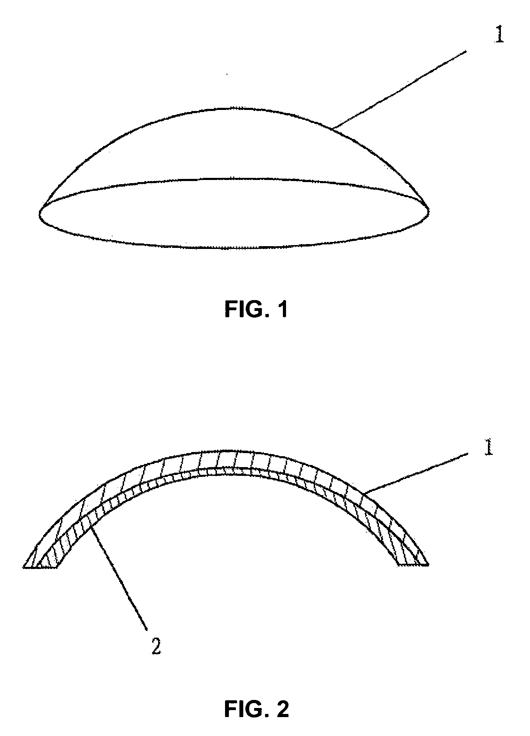

As shown in FIGS. 1 and 2, the biological artificial cornea comprises a substrate 1 prepared from an animal cornea by crosslinking and fixing with a non-aldehyde fixative and minimizing antigens. An active surface layer 2 is formed by coupling the inner (eyeball-facing) surface of substrate 1 with an active component consisting of a polypeptide or glycosaminoglycan capable of adhering to growth factors. One example of the polypeptide is the polypeptide obtained from the condensation of 16 lysines (K16), glycine (G), arginine (R), asparagic acid (D), serine (S), praline (P) and cysteine (C), and said glycosaminoglycan is hyaluronic acid, chondroitin sulfate, dermatan sulfate, heparin, acetylheparin sulfate or keratan sulfate. This biological artificial cornea can be made from the following steps:

1. Selection of materials: Fresh eyeballs are collected from healthy pigs and frozen in special preservation bottles before being transported.

2. Pretreatment: The animal corneas are excised and trimmed. The corneas are then placed in artificial tears or glycerol preservation solution and frozen at -18 C for 24 hours. Thereafter, the corneas are removed, thawed and soaked in a surfactant solution of Triton X-100, sodium cholate, hydroxymethyl aminomethane (Tris), sodium dodecyl sulfate (SDS) or CHAPS for 16-20 hours (or soaked in pepsin, trypsin or a mixed enzyme solution of the two for 2-4 hours), followed by washing, and if necessary, washing for 10-20 minutes with ultrasound.

3. Crosslinking fixation: The collagen molecules in the substrate 1 are crosslinked and fixed at room temperature for 8-48 hours with an epoxide fixative solution.

4. Minimizing antigens: The specific active group, namely -OH or -NH₂ or -SH, in the proteins of the substrate 1 is blocked with an active reagent such as an acid anhydride or methylating agent or epoxide, and the specific hydrogen bonding in the spiral chains of the proteins in the substrate 1 is replaced using a reagent with strong hydrogen bonding (e. g., guanidine hydrochloride solution) to alter the configuration.

5. Surface Modification: Active surface layer 2 is formed by coupling the substrate surface 1 with the polypeptide obtained from the condensation of 16 lysines (K16), glycine (G), arginine (R), asparagic acid (D), serine(S), praline (P) and cysteine (C), and a glycosaminoglycan, using a coupling agent.

6. Packaging: The product is sterilized with a sterilizing agent and packed and sealed in a small bottle filled with preservation solution under aseptic conditions.

While the description above refers to particular embodiments of the present invention, it will be understood that many modifications may be made without departing from the spirit thereof. The accompanying claims are intended to cover such modifications as would fall within the true scope and spirit of the present invention.

## Claims

1. A method for preparing an artificial cornea for implantation into a human body, comprising:
providing a natural animal cornea as a substrate;
crosslinking and fixing the substrate with a non-aldehyde fixative;
minimizing the antigens from the substrate; and
coupling an active layer to the substrate,
wherein minimizing the antigens from the substrate includes:
utilizing an active reagent selected from low molecular weight organic acid anhydride, acyl chloride, or acylamide, to block specific active groups, such as -OH, -NH₂, -SH, etc., in the proteins of the substrate; and
utilizing a reagent with strong hydrogen bonding power, being a guanidine compound, to replace the specific hydrogen bonding in the spiral chains of the protein molecules in the substrate and alter its specific configuration.

2. The method of claim 1, wherein the active layer includes a polypeptide.

3. The method of claim 1, wherein the cross-linking and fixing step is accomplished using an epoxy compound, a diamide, a diisocyanate, or a carbodiimide, and wherein the epoxy compound is preferably an epoxide that has a hydrocarbon backbone, that is water-soluble, and which does not contain an ether or ester linkage in its backbone.

4. An artificial cornea for implantation into a human body, comprising: a natural animal cornea as a substrate that has been crosslinked, and from which antigens have been minimized, the substrate having an active layer coupled thereto, wherein minimizing the antigens from the substrate includes:
utilizing an active reagent selected from low molecular weight organic acid anhydride, acyl chloride, or acylamide, to block apecific active groups, such as -OH, -NH₂, -SH, etc., in the proteins of the substrate; and
utilizing a reagent with strong hydrogen bonding power, being a guanidine compound, to replace the specific hydrogen bonding in the spiral chains of the protein molecules in the substrate and alter its specific configuration.

5. The cornea of claim 4, wherein the active layer includes a polypeptide.

6. The cornea of claim 4, wherein the substrate is fixed by an epoxy compound, a diamide, a diisocyanate, or a carbodiimide, and wherein the epoxy compound is preferably an epoxide that has a hydrocarbon backbone, that is water-soluble, and which does not contain an ether or ester linkage in its backbone.

7. A cornea for implantation into a human body, the cornea made by a method comprising:
providing a natural animal cornea as a substrate;
crosslinking and fixing the substrate;
minimizing the antigens from the substrate; and
coupling an active layer to the substrate,
wherein minimizing the antigens from the substrate includes:
utilizing an active reagent selected from low molecular weight organic acid anhydride, acyl chloride, or acylamide, to block apecific active groups, such as -OH, -NH₂, -SH, etc., in the proteins of the substrate; and
utilizing a reagent with strong hydrogen bonding power, being a guanidine compound, to replace the specific hydrogen bonding in the spiral chains of the protein molecules in the substrate and alter its specific configuration.

8. The cornea of claim 7, wherein the active layer includes a polypeptide.

9. The cornea of claim 7, wherein the substrate is fixed by an epoxy compound, a diamide, a diisocyanate, or a carbodiimide, and wherein the epoxy compound is preferably an epoxide that has a hydrocarbon backbone, that is water-soluble, and which does not contain an ether or ester linkage in its backbone.

## Patentansprüche

1. Verfahren zur Herstellung einer künstlichen Hornhaut zur Implantation in einen menschlichen Körper, welches Folgendes umfasst:
Bereitstellen einer natürlichen tierischen Hornhaut als ein Substrat;
Vernetzen und Fixieren des Substrates mit einem Nicht-Aldehyd-Fixiermittel;
Minimieren der Antigene aus dem Substrat; und
Koppeln einer aktiven Schicht an das Substrat,
wobei das Minimieren der Antigene aus dem Substrat Folgendes beinhaltet:
Verwenden eines aktiven Reagens ausgewählt aus organischem Säureanhydrid mit niedrigem Molekulargewicht, Acylchlorid oder Acylamid zum Blockieren spezifischer aktiver Gruppen, wie -OH, -NH₂, -SH usw., in den Proteinen des Substrates; und
Verwenden eines Reagens mit starker Wasserstoffbindungskraft, bei dem es sich um eine Guanidin-Verbindung handelt, zum Ersetzen der spezifischen Wasserstoffbindung in den Helices der Proteinmoleküle in dem Substrat und Verändern seiner spezifischen Konfiguration.

2. Verfahren nach Anspruch 1, wobei die aktive Schicht ein Polypeptid beinhaltet.

3. Verfahren nach Anspruch 1, wobei der Vernetzungs- und Fixierungsschritt mit Hilfe einer Epoxy-Verbindung, eines Diamids, eines Diisocyanats oder eines Carbodiimids erreicht wird, und wobei die Epoxy-Verbindung vorzugsweise ein Epoxid ist, welches eine Kohlenwasserstoffhauptkette aufweist, die wasserlöslich ist, und welches keine Ether- oder Esterbindung in seiner Hauptkette enthält.

4. Künstliche Hornhaut zur Implantation in einen menschlichen Körper, welche Folgendes umfasst: eine natürliche tierische Hornhaut als ein Substrat, das vernetzt wurde, und aus welcher Antigene minimiert wurden, wobei das Substrat eine aktive Schicht daran gekoppelt aufweist, wobei das Minimieren der Antigene aus dem Substrat Folgendes beinhaltet:
Verwenden eines aktiven Reagens ausgewählt aus organischem Säureanhydrid mit niedrigem Molekulargewicht, Acylchlorid oder Acylamid zum Blockieren spezifischer aktiver Gruppen, wie -OH, -NH₂, -SH usw., in den Proteinen des Substrates; und
Verwenden eines Reagens mit starker Wasserstoffbindungskraft, wobei es sich um eine Guanidin-Verbindung handelt, zum Ersetzen der spezifischen Wasserstoffbindung in den Helices der Proteinmoleküle in dem Substrat und Verändern seiner spezifischen Konfiguration.

5. Hornhaut nach Anspruch 4, wobei die aktive Schicht ein Polypeptid beinhaltet.

6. Hornhaut nach Anspruch 4, wobei das Substrat durch eine Epoxy-Verbindung, ein Diamid, ein Diisocyanat oder ein Carbodiimid fixiert ist, und wobei die Epoxy-Verbindung vorzugsweise ein Epoxid ist, welches eine Kohlenwasserstoffhauptkette aufweist, die wasserlöslich ist, und welches keine Ether- oder Esterbindung in seiner Hauptkette enthält.

7. Hornhaut zur Implantation in einen menschlichen Körper, wobei die Hornhaut durch ein Verfahren hergestellt ist, welches Folgendes umfasst:
Bereitstellen einer natürlichen tierischen Hornhaut als ein Substrat;
Vernetzen und Fixieren des Substrates;
Minimieren der Antigene aus dem Substrat; und
Koppeln einer aktiven Schicht an das Substrat,
wobei das Minimieren der Antigene aus dem Substrat Folgendes beinhaltet:
Verwenden eines aktiven Reagens ausgewählt aus organischem Säureanhydrid mit niedrigem Molekulargewicht, Acylchlorid oder Acylamid zum Blockieren spezifischer aktiver Gruppen, wie -OH, -NH₂, -SH usw., in den Proteinen des Substrates; und
Verwenden eines Reagens mit starker Wasserstoffbindungskraft, bei dem es sich um eine Guanidin-Verbindung handelt, zum Ersetzen der spezifischen Wasserstoffbindung in den Helices der Proteinmoleküle in dem Substrat und Verändern seiner spezifischen Konfiguration.

8. Hornhaut nach Anspruch 7, wobei die aktive Schicht ein Polypeptid beinhaltet.

9. Hornhaut nach Anspruch 7, wobei das Substrat durch eine Epoxy-Verbindung, ein Diamid, ein Diisocyanat oder ein Carbodiimid fixiert ist, und wobei die Epoxy-Verbindung vorzugsweise ein Epoxid ist, welches eine Kohlenwasserstoffhauptkette aufweist, die wasserlöslich ist, und welches keine Ether- oder Esterbindung in seiner Hauptkette enthält.

## Revendications

1. Procédé de préparation d'une cornée artificielle destinée à être implantée dans un corps humain, ledit procédé comprenant :
la fourniture d'une cornée animale naturelle en tant que substrat ;
la réticulation et la fixation du substrat avec un fixateur non aldéhydique ;
la minimisation des antigènes du substrat ; et
le couplage d'une couche active au substrat,
dans lequel la minimisation des antigènes du substrat comprend :
l'utilisation d'un réactif actif choisi parmi un anhydride d'acide organique de faible masse moléculaire, le chlorure d'acyle, ou l'acylamide, pour bloquer les groupes actifs spécifiques, tels que -OH, -NH₂, -SH, etc., dans les protéines du substrat ; et
l'utilisation d'un réactif avec une forte capacité de liaison hydrogène, étant un composé guanidine, pour remplacer la liaison hydrogène spécifique dans les chaînes spiralées des molécules de protéines dans le substrat et modifier sa configuration spécifique.

2. Procédé selon la revendication 1, dans lequel la couche active comprend un polypeptide.

3. Procédé selon la revendication 1, dans lequel l'étape de réticulation et de fixation est réalisée en utilisant un composé époxy, un diamide, un diisocyanate, ou un carbodiimide, et dans lequel le composé époxy est de préférence un époxyde qui a un squelette hydrocarboné, qui est soluble dans l'eau, et qui ne contient pas de liaison éther ou ester dans son squelette.

4. Cornée artificielle destinée à être implantée dans un corps humain, ladite cornée comprenant : une cornée animale naturelle en tant que substrat qui a été réticulé, et duquel les antigènes ont été minimisés, une couche active étant couplée au dit substrat, dans laquelle la minimisation des antigènes du substrat comprend :
l'utilisation d'un réactif actif choisi parmi un anhydride d'acide organique de faible masse moléculaire, le chlorure d'acyle, ou l'acylamide, pour bloquer les groupes actifs spécifiques, tels que -OH, -NH₂, -SH, etc., dans les protéines du substrat ; et
l'utilisation d'un réactif avec une forte capacité de liaison hydrogène, étant un composé guanidine, pour remplacer la liaison hydrogène spécifique dans les chaînes spiralées des molécules de protéines dans le substrat et modifier sa configuration spécifique.

5. Cornée selon la revendication 4, dans laquelle la couche active comprend un polypeptide.

6. Cornée selon la revendication 4, dans laquelle le substrat est fixé par un composé époxy, un diamide, un diisocyanate, ou un carbodiimide, et dans laquelle le composé époxy est de préférence un époxyde qui a un squelette hydrocarboné, qui est soluble dans l'eau, et qui ne contient pas de liaison éther ou ester dans son squelette.

7. Cornée destinée à être implantée dans un corps humain, la cornée étant fabriquée par un procédé comprenant :
la fourniture d'une cornée animale naturelle en tant que substrat ;
la réticulation et la fixation du substrat ;
la minimisation des antigènes du substrat ; et
le couplage d'une couche active au substrat,
dans lequel la minimisation des antigènes du substrat comprend :
l'utilisation d'un réactif actif choisi parmi un anhydride d'acide organique de faible masse moléculaire, le chlorure d'acyle, ou l'acylamide, pour bloquer les groupes actifs spécifiques, tels que -OH, -NH₂, -SH, etc., dans les protéines du substrat ; et
l'utilisation d'un réactif avec une forte capacité de liaison hydrogène, étant un composé guanidine, pour remplacer la liaison hydrogène spécifique dans les chaînes spiralées des molécules de protéines dans le substrat et modifier sa configuration spécifique.

8. Cornée selon la revendication 7, dans laquelle la couche active comprend un polypeptide.

9. Cornée selon la revendication 7, dans laquelle le substrat est fixé par un composé époxy, un diamide, un diisocyanate, ou un carbodiimide, et dans laquelle le composé époxy est de préférence un époxyde qui a un squelette hydrocarboné, qui est soluble dans l'eau, et qui ne contient pas de liaison éther ou ester dans son squelette.
